# EUROPÄISCHE PATENTSCHRIFT

(11) **EP 0 750 606 B1**
(45) Veröffentlichungstag und Bekanntmachung des Hinweises auf die Patenterteilung: **28.10.1998**
(21) Anmeldenummer: 95911314.3
(22) Anmeldetag: 09.03.1995
(51) Int. Cl.: C07C 213/06, C11D 1/62, A61K 7/075

(54) **VERFAHREN ZUR HERSTELLUNG VON ESTERQUATS**
PROCESS FOR PRODUCING ESTERQUATS
PROCEDE DE PRODUCTION D'ESTERS QUATERNAIRES

(30) Priorität: 18.03.1994 DE 4409322
(43) Veröffentlichungstag der Anmeldung: 02.01.1997
(73) Patentinhaber: Henkel Kommanditgesellschaft auf Aktien, 40191 Düsseldorf (DE)
(72) Erfinder: PI SUBIRANA, Rafael, E-08400 Granollers (ES); BIGORRA LLOSAS, Joaquim, E-08203 Sabadell (ES); TRIUS OLIVA, Antonio, E-Valldoreix (ES)
(86) Internationale Anmeldenummer: EP9500861
(87) Internationale Veröffentlichungsnummer: WO9525713

(56) Entgegenhaltungen:
- EP-A- 0 498 050
- EP-A- 0 525 271
- EP-A- 0 550 361
- WO-A-91/01295

## Beschreibung

Die Erfindung betrifft ein Verfahren zur Herstellung von Esterquats, bei dem man Fettsäureglycerinester auf direktem Wege mit tertiären Hydroxyaminen umestert und anschließend quaterniert.

### Stand der Technik

Quaternierte Fettsäuretriethanolaminestersalze, sogenannte "Esterquats", haben in den letzten Jahren als ökotoxikologisch unbedenkliche Rohstoffe für Wäscheweichspüler zunehmend an Bedeutung gewonnen [vgl. O.Ponsati in **C.R. CED-Kongress, Barcelona, 167 (1992)** und R.Puchta in **C.R. CED-Kongress, Sitges, 59 (1993)]**. Gemäß der Lehre der Internationale Patentanmeldung **WO 91101295** (Henkel) geht man zur Herstellung von Esterquats üblicherweise von Triethanolamin oder Ethylenoxid-Addukten an Triethanolamin aus, die zunächst in Gegenwart von unterphosphoriger Säure mit linearen C_{16/18}-Fettsäuren partiell verestert und dann mit Luft behandelt werden. Anschließend erfolgt die Quaternierung der Fettsäuretriethanolaminester beispielsweise mit Alkylhalogeniden oder vorzugsweise Dialkylsulfaten. Aus ökonomischen Gründen ist es wünschenswert, als Ausgangsstoffe für die Herstellung von Esterquats anstelle der Fettsäuren unmittelbar natürliche Fette und Öle einzusetzen, aus denen diese gewonnen werden. Nach Untersuchungen der Anmelderin hat einer technischen Realisierung jedoch bislang der Umstand im Wege gestanden, daß die Umesterung in Gegenwart der für diesen Reaktionstyp gebräuchlichen alkalischen Katalysatoren - Kaliumhydroxid oder Natriummethylat - mit zu langen Kesselbelegungszeiten verbunden ist und in der Regel zu verfärbten Produkten führt, die einer aufwendigen Nachbleiche unterworfen werden müssen. So ist beispielsweise aus der **EP 0550361 A1** ein Verfahren zur Herstellung von Esterquats bekannt, bei dem man die Umesterung von Triglyceriden mit Alkanolaminen in Gegenwart von Natriummethylat durchführt. Die nach der anschließenden Quaternierung erhaltenen Produkte sind jedoch gelblich verfärbt und müssen nachträglich einer Bleiche unterworfen werden.

Demzufolge hat die Aufgabe der Erfindung darin bestanden, ein Verfahren zur Herstellung von Esterquats auf Basis von Fetten und Ölen als Ausgangsstoffen zu Verfügung zu stellen, das frei von den geschilderten Nachteilen ist.

### Beschreibung der Erfindung

Gegenstand der Erfindung ist ein Verfahren zur Herstellung von Esterquats, bei dem man Fettsäureglycerinester in Gegenwart von Alkali- und/oder Erdalkaliborhydriden und hypophosphoriger Säure bzw. deren Alkali- und/oder Erdalkalisalzen sowie gegebenenfalls freier Fettsäure einer Umesterung mit hydroxyfunktionalisierten tertiären Aminen unterwirft und die resultierenden Produkte in an sich bekannter Weise quaterniert.

Überraschenderweise wurde gefunden, daß die Verwendung von Borhydriden gegenüber anderen, konventionellen Umesterungskatalysatoren zu einer raschen und vollständigen Umsetzung führt, bei der zudem Reaktionsprodukte erhalten werden, die sich durch eine besonders hohe Farbqualität auszeichnen. Das erfindungsgemäße Verfahren bietet darüber hinaus den Vorteil, daß anstelle von Fettsäuren unmittelbar Fette und Öle eingesetzt werden können, was die Wirtschaftlichkeit des Verfahrens erhöht.

### Esterquats

Unter der Bezeichnung Esterquats werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91101295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Stellvertretend für den umfangreichen Stand der Technik sei an dieser Stelle auf die Druckschriften **US 3,915,867, US 4,370,272, EP 0239910A2, EP 0293955 A2, EP 0295739 A2 und EP 0309052 A2** verwiesen.

Die quaternierten Fettsäuretriethanolaminestersalze folgen der Formel (I), 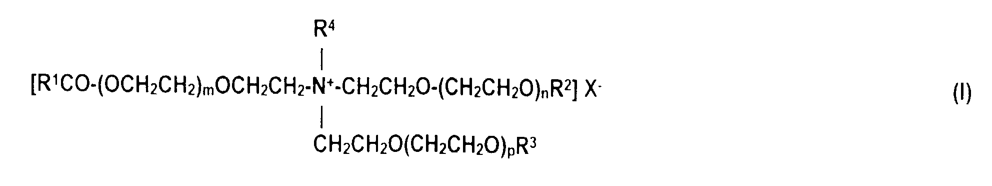 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H- Gruppe, m, n und p in Summe für 0 oder Zahlen von 1 bis 12, q für Zahlen von 1 bis 12 und X für Halogenid, Alkylsulfat oder Alkylphosphat steht. Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Aus anwendungstechnischer Sicht haben sich quaternierte Fettsäuretriethanolaminestersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel **(II)** in Betracht, 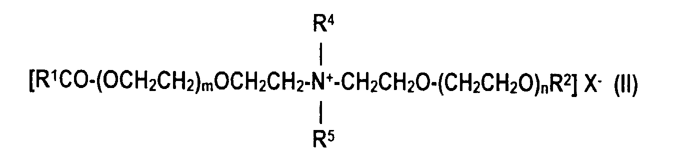 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Als weitere Gruppe geeigneter Esterquats sind schließlich die quaternierten Estersalze von Fettsäuren mit 1,2-Dihydroxypropyldialkylaminen der Formel **(III)** zu nennen, 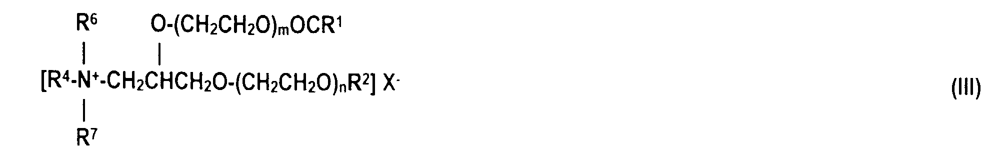 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht. Hinsichtlich der Auswahl der bevorzugten Fettsäuren gelten die für **(I)** genannten Beispiele auch für die Esterquats der Formeln **(II)** und **(III)**.

### Fettsäureglycerinester

Unter dem Begriff Fettsäureglycerinester sind synthetische oder vorzugsweise natürliche Triglyceride, Diglyceride, Monoglyceride oder deren technische Gemische zu verstehen. Die Glycerinester enthalten in der Regel Fettsäuren mit 6 bis 22 und vorzugsweise 12 bis 18 Kohlenstoffatomen. Sie können gesättigt sein oder bis zu 3 Doppelbindungen enthalten. Die Glycerinester können ferner ausschließlich einen Fettsäuretyp oder aber bis zu 3 verschiedene Fettsäuren enthalten. Typische Beispiele für Triglyceride, die im Sinne des erfindungsgemäßen Verfahrens in Betracht kommen, sind Palmöl, Palmkernöl, Kokosöl, Olivenöl, Rapsöl alter und neuer Züchtung, Sonnenblumenöl alter und neuer Züchtung, Baumwollsaatöl, Leinöl, Erdnußöl, Rindertalg und Schweineschmalz. Aus anwendungstechnischer Sicht ist der Einsatz von vollständig oder anteilig gehärtetem Rindertalg oder Palmöl in einem lodzahlbereich von 0 bis etwa 50 bevorzugt.

### Hydroxyfunktionalisierte tertiäre Amine

Bei der Umesterungsreaktion tritt mindestens eine Fettsäure des Glycerids mit einer OH-Gruppe eines hydroxyfunktionalisierten tertiären Amins zusammen. Typische Beispiele für geeignete Amine sind Diethanolalkylamine wie beispielsweise Diethanolmethylamin und 1,2-Dihydroxypropyldialkylamine wie beispielsweise 1,2-Dihydroxypropyldimethylamin sowie insbesondere Triethanolamin. Die Fettsäureglycerinester - berechnet auf der Grundlage der darin enthaltenen Fettsäuren - und die Amine können im molaren Verhältnis von 1,2:1 bis 2,2:1 und vorzugsweise 1,4 : 1 bis 1,9: 1 eingesetzt werden.

### Katalysatoren

Als Umesterungskatalysatoren kommen Alkali- und/oder Erdalkaliborhydride in Frage. Typische Beispiele sind Kalium- oder Magnesiumborhydrid und vorzugsweise Natriumborhydrid. Üblicherweise setzt man die Katalysatoren in Mengen von 50 bis 1000 und vorzugsweise 100 bis 500 ppm - bezogen auf die Glycerinester - ein. Als Co-Katalysatoren finden hypophosphorige Säure bzw. deren Alkali- und/ oder Erdalkalisalze, vorzugsweise aber Natriumhypophosphit, in Mengen von 0,01 bis 0,1 und vorzugsweise 0,05 bis 0,07 Gew.-% - bezogen auf die Glycerinester - Verwendung.

### Fettsäuren

In einer bevorzugten Ausführungsform des erfindungsgemäßen Verfahrens erfolgt die Umesterung unter Mitverwendung von freien Fettsäuren mit 6 bis 22, vorzugsweise 12 bis 18 Kohlenstoffatomen, die in Mengen von 1 bis 5 und insbesondere 2 bis 3 Gew.-% - bezogen auf den Glycerinester - eingesetzt werden können. Typische Beispiele für geeignete Fettsäuren sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, oder bei der Reduktion von Aldehyden aus der Roelen'schen Oxosynthese anfallen. Bevorzugt sind technische Fettsäuren mit 12 bis 18 Kohlenstoffatomen wie beispielsweise Kokos-, Palm-, Palmkern- oder Talgfettsäure, die mit der Fettsäure des eingesetzten Triglycerids korrespondieren.

### Umesterung

Die Umesterung wird üblicherweise bei Temperaturen im Bereich von 160 bis 240 und vorzugsweise 180 bis 220°C über einen Zeitraum von 1 bis 12, vorzugsweise 3 bis 6 h durchgeführt.

### Quaternierung

In der Quaternierung können die Fettsäurehydroxyalkylaminester und die Quaternierungsmittel im molaren Verhältnis von 1 : 0,95 bis 1:1,2, vorzugsweise 1:1 bis 1:1,1 eingesetzt werden. Als Alkylierungsmittel kommen Alylhalogenide wie beispielsweise Methylchlorid, Dialkylsulfate wie beispielsweise Dimethylsulfat, Dialkylphosphate und Dialkylcarbonate wie beispielsweise Dimethylcarbonat oder Diethylcarbonat in Betracht. Gewöhnlich findet die Umsetzung in einem Lösungsmittel, beispielsweise einem niederen Alkohol (Isopropylalkohol), einem Fettalkohol (Cetearethalkohol), einem nichtionischen Tensid (Alkyloligoglucosid, Anlagerungsprodukte von Ethylenoxid an Fettalkohole oder Partialglyceride) oder einer Hydroxycarbonsäure (Glycolsäure) statt. Vorzugsweise wird die Quaternierung bei Temperaturen im Bereich von 70 bis 100 und insbesondere 80 bis 90°C durchgeführt. Die Reaktionszeit kann 1 bis 24, vorzugsweise 2 bis 8 h betragen. Im Anschluß an die Quaternierung empfiehlt es sich, nicht umgesetztes Alkylierungsmittel durch Zugabe von Ammoniak, Glycin oder Monoethanolamin zu zerstören.

### Beispiele

**Allgemeine Herstellvorschrift.** In einem 1-1-Dreihalskolben mit Rührer, Innenthermometer und Destillationsaufsatz wurden 0,4 bis 0,63 Mol Triglycerid (entsprechend 1,2 bis 1,9 Mol bezogen auf Fettsäure), 149 g (1 mol) Triethanolamin, 0,05 Gew.-% Natriumhypophosphit, 100 bis 500 ppm Katalysator sowie gegebenenfalls 2,7 Gew.-% Talgfettsäure - alle Angaben bezogen auf Triglycerid - vorgelegt. Die Reaktionsmischung wurde über einen Zeitraum von 5 h auf eine Temperatur von 220°C erhitzt. Anschließend wurde der rohe Talgfettsäuretriethanolaminester abgekühlt, der Reaktionsansatz entspannt und unter ständigem Rühren innerhalb von 15 min 1 Liter Luft durchgeleitet. Die Ergebnisse der Veresterungsversuche sind in Tabelle 1 zusammengefaßt. In einem 1,5-I-Glasautoklaven mit Rührer und Innenthermometer wurden 0,5 Mol der zuvor hergestellten Ester in 150 ml Isopropylalkohol vorgelegt und mit 63 g (0,5 mol) Dimethylsulfat versetzt. Die Reaktionsmischung wurde 2 h bei 90°C gerührt, danach abgekühlt und entspannt. Um Spuren nicht umgesetzten Alkylierungsmittels zu zerstören wurde der Reaktionsansatz anschließend mit 2 g Glycin versetzt und über einen Zeitraum von 1 h bei 60°C gerührt.

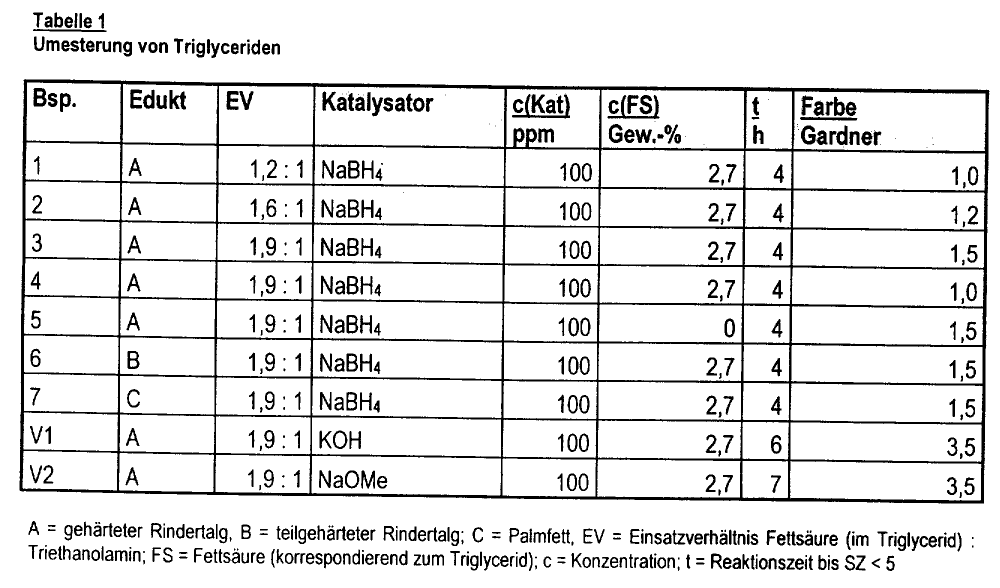

## Patentansprüche

1. Verfahren zur Herstellung von Esterquats, bei dem man Fettsäureglycerinester in Gegenwart von Alkali- und/oder Erdalkaliborhydriden und hypophosphoriger Säure bzw. deren Alkali- und/oder Erdalkalisalzen sowie gegebenenfalls freier Fettsäure einer Umesterung mit hydroxyfunktionalisierten tertiären Aminen unterwirft und die resultierenden Produkte in an sich bekannter Weise quaterniert.

2. Verfahren nach Anspruch 1, **dadurch gekennzeichnet**, daß man Esterquats der Formel **(I)** einsetzt, 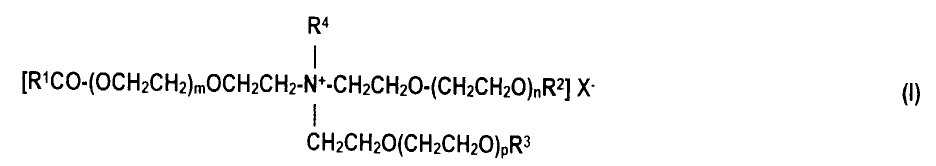 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Verfahren nach Anspruch 1, **dadurch gekennzeichnet,** daß man Esterquats der Formel **(II)** einsetzt, 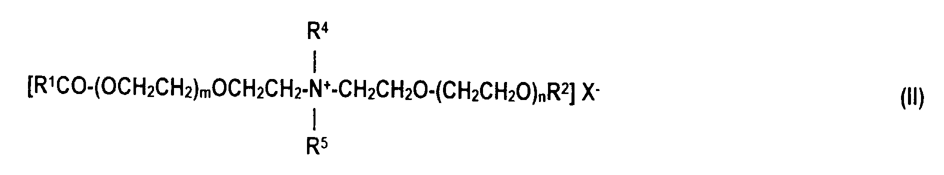 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Verfahren nach Anspruch 1, **dadurch gekennzeichnet**, daß man Esterquats der Formel **(III)** einsetzt, 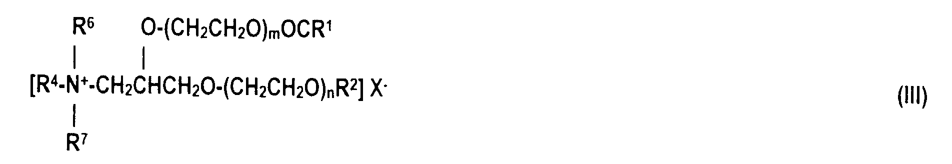 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verfahren nach den Ansprüchen 1 bis 4, **dadurch gekennzeichnet**, daß man als Fettsäureglycerinester, Triglyceride, Diglyceride, Monoglyceride oder deren technische Gemische einsetzt.

6. Verfahren nach den Ansprüchen 1 bis 5, **dadurch gekennzeichnet**, daß man als Fettsäureglycerinester gegebenenfalls vollständig oder anteilig gehärteten Rindertalg oder Palmöl in einem lodzahlbereich von 0 bis etwa 50 einsetzt.

7. Verfahren nach den Ansprüchen 1 bis 6, **dadurch gekennzeichnet**, daß man als hydroxyfunktionalisierte tertiäre Amine Triethanolamin, Diethanolalkylamine und/oder 1,2- Dihydroxypropyldialkylamine einsetzt.

8. Verfahren nach den Ansprüchen 1 bis 7, **dadurch gekennzeichnet**, daß man die Fettsäureglycerinester - berechnet auf der Grundlage der darin enthaltenen Fettsäuren - und die Amine im molaren Verhältnis von etwa 1,2:1 bis 2,2:1 einsetzt.

9. Verfahren nach den Ansprüchen 1 bis 8, **dadurch gekennzeichnet**, daß man als Katalysator Natriumborhydrid einsetzt.

10. Verfahren nach den Ansprüchen 1 bis 9, **dadurch gekennzeichnet**, daß man die Katalysatoren in Mengen von 50 bis 1000 ppm - bezogen auf die Glycerinester - einsetzt.

11. Verfahren nach den Ansprüchen 1 bis 10, **dadurch gekennzeichnet**, daß man die hypophosphorige Säure bzw. deren Salze in Mengen von 0,01 bis 0,1 Gew.-% - bezogen auf die Glycerinester - einsetzt.

12. Verfahren nach den Ansprüchen 1 bis 11, **dadurch gekennzeichnet**, daß man Fettsäuren mit 6 bis 22 Kohlenstoffatomen in Mengen von 1 bis 5 Gew.-% - bezogen auf die Glycerinester - einsetzt.

13. Verfahren nach den Ansprüchen 1 bis 12, **dadurch gekennzeichnet**, daß man die Umesterung bei Temperaturen im Bereich von 120 bis 180°C über einen Zeitraum von 1 bis 12 h durchführt.

14. Verfahren nach den Ansprüchen 1 bis 12, **dadurch gekennzeichnet**, daß man Quaternierungsmittel einsetzt, die ausgewählt sind aus der Gruppe, die gebildet wird von Alkylhalogeniden, Dialkylsulfaten, Dialkylphosphaten und Dialkylcarbonaten.

## Claims

1. A process for the production of esterquats, in which fatty acid glycerol esters are subjected to transesterification with hydroxyfunctionalized tertiary amines in the presence of alkali metal and/or alkaline earth metal borohydrides and hypophosphorous acid or alkali metal and/or alkaline earth metal salts thereof and, optionally, free fatty acid and the resulting products are quaternized in known manner.

2. A process as claimed in claim 1, characterized in that the esterquats correspond to formula **(I)**: 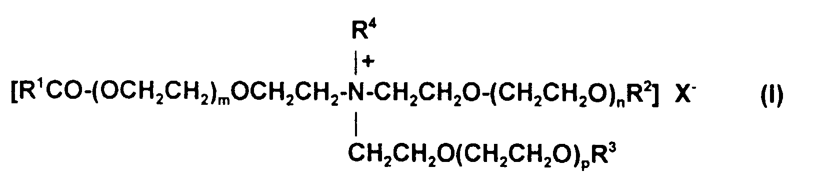 in which R¹CO is an acyl radical containing 6 to 22 carbon atoms, R² and R³ independently of one another represent hydrogen or have the same meaning as R¹CO, R⁴ is an alkyl radical containing 1 to 4 carbon atoms or a (CH₂-CH₂O)_{q}H group, m, n and p have a combined value of 0 or 1 to 12, q is a number of 1 to 12 and X is halide, alkyl sulfate or alkyl phosphate.

3. A process as claimed in claim 1, characterized in that the esterquats correspond to formula **(II)**: 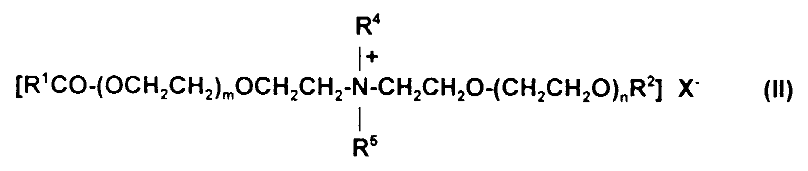 in which R¹CO is an acyl radical containing 6 to 22 carbon atoms, R² is hydrogen or has the same meaning as R¹CO, R⁴ and R⁵ independently of one another represent alkyl radicals containing 1 to 4 carbon atoms, m and n have a combined value of 0 or 1 to 12 and X stands for halide, alkyl sulfate or alkyl phosphate.

4. A process as claimed in claim 1, characterized in that the esterquats correspond to formula **(III)**: 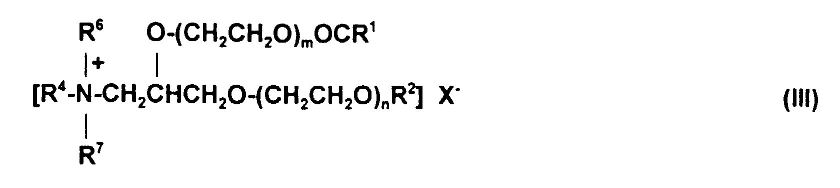 in which R¹CO is an acyl radical containing 6 to 22 carbon atoms, R² is hydrogen or has the same meaning as R¹CO, R⁴, R⁶ and R⁷ independently of one another represent alkyl radicals containing 1 to 4 carbon atoms, m and n have a combined value of 0 or 1 to 12 and X stands for halide, alkyl sulfate or alkyl phosphate.

5. A process as claimed in claims 1 to 4, characterized in that triglycerides, diglycerides, monoglycerides or technical mixtures thereof are used as the fatty acid glycerol esters.

6. A process as claimed in claims 1 to 5, characterized in that completely or partly hydrogenated beef tallow or palm oil in an iodine value range of 0 to about 50 is used as the fatty acid glycerol ester.

7. A process as claimed in claims 1 to 6, characterized in that triethanolamine, diethanol alkylamines and/or 1,2-dihydroxypropyl dialkylamines are used as the hydroxyfunctionalized tertiary amines.

8. A process as claimed in claims 1 to 7, characterized in that the fatty acid glycerol esters - expressed on the basis of the fatty acids present in them - and the amines are used in a molar ratio of about 1.2:1 to 2.2:1.

9. A process as claimed in claims 1 to 8, characterized in that sodium borohydride is used as the catalyst.

10. A process as claimed in claims 1 to 9, characterized in that the catalysts are used in quantities of 50 to 1000 ppm, based on the glycerol ester.

11. A process as claimed in claims 1 to 10, characterized in that in the hypophosphorous acid or its salt is used in a quantity of 0.01 to 0.1% by weight, based on the glycerol ester.

12. A process as claimed in claims 1 to 11, characterized in that fatty acids containing 6 to 22 carbon atoms are used in quantities of 1 to 5% by weight, based on the glycerol ester.

13. A process as claimed in claims 1 to 12, characterized in that the transesterification is carried out over a period of 1 to 12 h at temperatures of 120 to 180°C.

14. A process as claimed in claims 1 to 12, characterized in that quaternizing agents selected from the group consisting of alkyl halides, dialkyl sulfates, dialkyl phosphates and dialkyl carbonates are used.

## Revendications

1. Procédé de production d'esterquats, dans lequel on soumet des esters de glycérol et d'acide gras à une transestérification avec des amines tertiaires fonctionnalisés par un hydroxyle, en présence de borohydrures de métal alcalin et/ou alcalino-terreux et d'acide hypophosphoreux ou de ses sels de métal alcalin et/ou alcalino-terreux ainsi que le cas échéant d'acide gras libre et on quaternise les produits résultants d'une manière connue en soi.

2. Procédé selon la revendication 1,
caractérisé en ce qu'
on met en oeuvre des esterquats de formule (I) 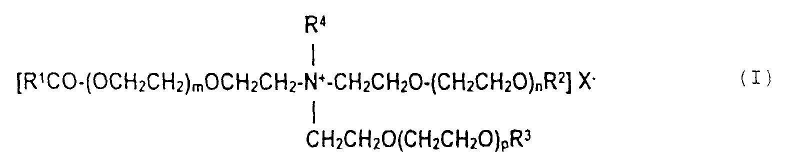 dans laquelle R¹CO représente un radical acyle ayant de 6 à 22 atomes de carbone, R² et R³ indépendamment l'un de l'autre, représentent de l'hydrogène ou R¹ CO, R⁴ représente un radical alkyle ayant de 1 à 4 atomes de carbone ou un groupe (CH₂CH₂O)_{q}H, m, n et p globalement représentent 0 ou des nombres allant de 1 à 12, q représente des nombres allant de 1 à 12 et x représente un halogénure, un alkylsulfate ou un alkylphosphate.

3. Procédé selon la revendication 1,
caractérisé en ce qu'
on met en oeuvre des esterquats de formule (II) 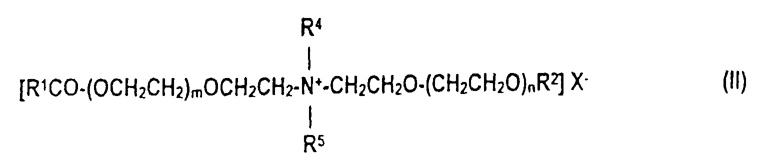 dans laquelle R¹CO représente un radical acyle ayant de 6 à 22 atomes de carbone, R² représente de l'hydrogène ou R¹CO, R⁴ et R⁵, indépendamment l'un de l'autre, représentent des radicaux alkyle ayant de 1 à 4 atomes de carbone, m et n au total représentent 0 ou des nombres allant de 1 à 12 et x représente un halogénure, un alkylsulfate ou un alkylphosphate.

4. Procédé selon la revendication 1,
caractérisé en ce qu'
on met en oeuvre des esterquats de formule (III) 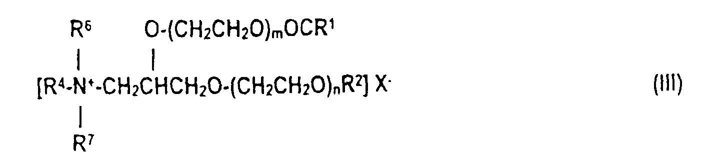 dans laquelle R¹CO représente un radical acyle ayant de 6 à 22 atomes de carbone, R² représente un hydrogène ou R¹CO, R⁴ , R⁶ et R⁷ indépendamment l'un de l'autre, représentent des radicaux alkyle ayant de 1 à 4 atomes de carbone, m et n au total représentent 0 ou des nombres allant de 1 à 12 et x représente un halogénure, un alkylsulfate ou un alkylphosphate.

5. Procédé selon les revendications 1 à 4,
caractérisé en ce qu'
on met en oeuvre comme ester de glycérol d'acide gras, des triglycérides, des diglycérides, ou des monoglycérides ou leurs mélanges industriels.

6. Procédé selon les revendications 1 à 5,
caractérisé en ce qu'
on met en oeuvre comme ester de glycérol d'acide gras, du suif de bovin, le cas échéant complètement ou partiellement durci ou de l'huile de palme dans une zone d'indice d'iode allant de 0 à environ 50.

7. Procédé selon les revendications 1 à 6,
caractérisé en ce qu'
on met en oeuvre comme amine tertiaire fonctionnalisée par un hydroxyle, de la triéthanolamine, de la diéthanolamine et/ou de la 1,2-dihydroxypropyldialkylamine.

8. Procédé selon les revendications 1 à 7,
caractérisé en ce qu'
on met en oeuvre les esters de glycérol d'acide gras, calculés sur la base des acides gras qui y sont contenus, et les amines, dans un rapport molaire allant d'environ 1,2:1 à 2,2:1.

9. Procédé selon les revendications 1 à 8,
caractérisé en ce qu'
on met en oeuvre comme catalyseur, du borohydrure de sodium.

10. Procédé selon les revendications 1 à 9,
caractérisé en ce qu'
on met en oeuvre les catalyseurs dans des quantités allant de 50 à 1000 ppm, rapportées aux esters de glycérol.

11. Procédé selon les revendications 1 à 10,
caractérisé en ce qu'
on met en oeuvre l'acide hypophosphoreux ou ses sels, en quantités allant de 0,01 à 0,1 % en poids rapporté aux esters de glycérol.

12. Procédé selon les revendications 1 à 11,
caractérisé en ce qu'
on met en oeuvre des acides gras ayant de 6 à 22 atomes de carbone en quantités allant de 1 à 5 % en poids, rapportées aux esters de glycérol.

13. Procédé selon les revendications 1 à 12,
caractérisé en ce qu'
on effectue la transestérification à des températures dans la zone allant de 120 à 180°C pendant un laps de temps de 1 à 12 h.

14. Procédé selon les revendications 1 à 12,
caractérisé en ce qu'
on met en oeuvre des agents de quaternisation, qui sont choisis dans le groupe qui est formé des halogénures d'alkyle, des sulfates de dialkyle, des phosphates de dialkyle et des carbonates de dialkyle.
